# FASCICULE DE BREVET EUROPEEN

(11) **EP 2 601 917 B1**
(45) Date de publication et mention de la délivrance du brevet: **29.10.2014**
(21) Numéro de dépôt: 12196078.5
(22) Date de dépôt: 07.12.2012
(51) Int. Cl.: A61F 9/007, A61F 9/009, A61N 7/02, A61N 7/00

(54) **Systeme et procede pour la determination de taille optimale d'un dispositif de generation d'ultrasons**
System und Verfahren zur Bestimmung der optimalen Größe einer Ultraschallerzeugungsvorrichtung
System and method for determining the optimal size of a device for generating ultrasound

(30) Priorité: 08.12.2011 FR 1161309
(43) Date de publication de la demande: 12.06.2013
(73) Titulaire: Eye Tech Care, 69140 rillieux-la-Pape (FR)
(72) Inventeur: Romano, Fabrizio, 01700 BEYNOST (FR); Aptel, Florent, 54520 LAXOU (FR); Farcy, Laurent, 69400 LIERGUES (FR)
(74) Mandataire: Regimbeau

(56) Documents cités:
- EP-A1- 2 092 916
- APTEL FLORENT ET AL: "Miniaturized high-intensity focused ultrasound device in patients with glaucoma: a clinical pilot study", INVESTIGATIVE OPHTHALMOLOGY & VISUAL SCIENCE, ASSOCIATION FOR RESEARCH IN VISION AND OPHTHALMOLOGY, vol. 52, no. 12, 1 novembre 2011 (2011-11-01), pages 8747-8753, XP002681265, ISSN: 1552-5783

## Description

### DOMAINE DE L'INVENTION

La présente invention concerne le domaine technique général du traitement non invasif d'une pathologie oculaire.

Elle concerne plus particulièrement un système et un procédé permettant de déterminer la taille optimale d'une sonde d'un dispositif de génération d'ultrasons focalisés de haute intensité pour le traitement d'une telle pathologie oculaire tel qu'un glaucome.

### ARRIERE-PLAN DE L'INVENTION

Le glaucome est une neuropathie optique, c'est à dire un trouble du nerf optique, due à une pression intraoculaire (PIO) élevée.

L'oeil est une structure creuse composée de deux segments: le segment antérieur entre la cornée et le cristallin, et le segment postérieur entre le cristallin et la rétine. Le segment antérieur contient un liquide transparent appelé "l'humeur aqueuse."

L'humeur aqueuse est formée dans la chambre postérieure du segment antérieur de l'oeil par le corps ciliaire. Le liquide, qui est généré à un taux relativement constant, passe ensuite autour du cristallin, à travers l'ouverture pupillaire de l'iris et dans la chambre antérieure de l'oeil. L'humeur aqueuse est ensuite évacuée principalement à travers le trabéculum et le canal de Schlemm.

Lorsque l'humeur aqueuse n'est plus évacuée suffisamment rapidement, celle-ci s'accumule, ce qui induit une augmentation de la PIO. L'augmentation de la PIO compresse les axones dans le nerf optique et peut également compromettre la vascularisation du nerf optique. Une PIO élevée pendant une longue période peut induire une perte de vision totale.

La seule approche thérapeutique actuellement disponible pour traiter le glaucome consiste à réduire la pression intraoculaire :
- soit en améliorant le drainage d'humeur aqueuse,
- soit en réduisant la production d'humeur aqueuse.

On connaît du document WO 2009/103721 un dispositif pour réduire la production d'humeur aqueuse basé sur le principe de cyclo coagulation par Ultrasons Focalisés de Haute intensité qui consiste à détruire une partie des corps ciliaires afin de réduire la production d'humeur aqueuse.

Le dispositif décrit dans WO 2009/103721 permet le traitement du glaucome en un seul geste.

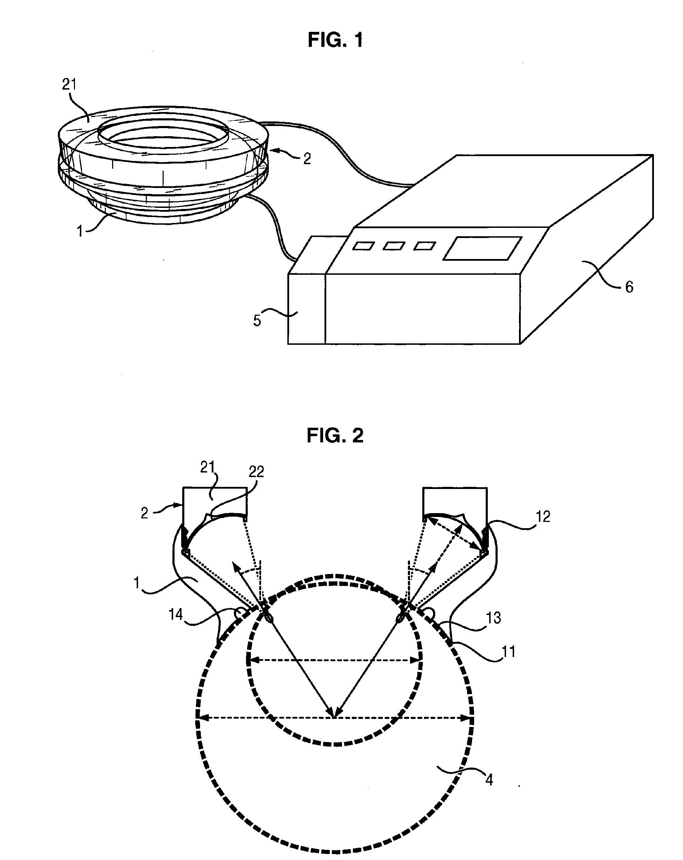

En référence aux figures 1 et 2, ce dispositif 1 comprend une sonde composée d'un anneau et de moyens de génération d'ultrasons. L'anneau présente une partie proximale destinée à être en contact avec un oeil et une partie distale destinée à recevoir les moyens de génération d'ultrasons focalisés de haute intensité. Les moyens de génération d'ultrasons comprennent six transducteurs à profil concave en forme en segment de cylindre positionnés sur une couronne cylindrique.

Les dimensions de l'anneau doivent être adaptées en fonction des dimensions de l'oeil du patient à traiter pour permettre un positionnement précis des moyens de génération d'ultrasons de sorte à détruire de manière sélective et précise une partie du corps ciliaire et d'épargner les structures adjacentes.

On connaît du document intitulé *« miniaturized High-Intensity Focused Ultrasound Device in Patients with Glaucoma : A Clinical Pilot Study »*, une méthode permettant de choisir un modèle de sonde parmi une pluralité de modèles de sonde. Pour choisir le modèle de sonde le plus adapté au traitement d'un oeil, ce document propose l'utilisation d'une image par biomicroscopie ultrason.

Un inconvénient de cette méthode est qu'elle nécessite l'utilisation d'un dispositif d'imagerie par biomicroscopie ultrasonore, dispositif peu répandu et coûteux.

Un but de la présente invention est de proposer un système et un procédé permettant de déterminer les dimensions optimales de la sonde du dispositif décrit dans WO 2009/103721 en fonction des dimensions de l'oeil du patient, ce système et ce procédé ne nécessitant pas l'utilisation d'un dispositif d'imagerie coûteux.

### BREVE DESCRIPTION DE L'INVENTION

A cet effet, l'invention propose un procédé pour la détermination d'un modèle optimal de sonde de traitement d'une pathologie oculaire parmi une pluralité de modèles de sondes différents, chaque sonde comportant un anneau de forme conique incluant une extrémité proximale destinée à être en contact avec un oeil d'un patient et une extrémité distale destinée à recevoir des moyens de génération d'ultrasons, le procédé comprenant les étapes suivantes :
- la réception d'au moins une mesure d'au moins un paramètre biométrique de l'oeil,
- la détermination du modèle optimal de sonde en fonction dudit et au moins un paramètre biométrique.

Les modèles de sondes de la pluralité de modèles de sondes peuvent être différents en ce qu'ils comportent des anneaux de dimensions différentes.

On entend, dans le cadre de la présente invention, par *« paramètre biométrique de l'oeil »*, une dimension d'une partie de l'oeil telle que :
- la largeur (i.e. le diamètre) de l'iris,
- la longueur axiale de l'oeil,
- le rayon de courbure de la sclérotique,
- l'épaisseur de la cornée transparente,
- le rayon antérieur ou postérieur du cristallin,
- le diamètre du cristallin, l'épaisseur du cristallin.

Dans tous les cas, la (ou les) mesure(s) reçue(s) est (sont) dépourvue(s) d'image par biomicroscopie ultrasonore de l'oeil à traiter. En d'autres termes, cette (ces) mesure(s) ne contient (contiennent) pas d'image par biomicroscopie ultrasonore de l'oeil à traiter.

La détermination d'un modèle optimal de sonde peut comprendre le choix parmi une pluralité de modèles de sonde, du modèle de sonde le plus adapté au traitement à mettre en oeuvre. Ce choix est fonction du (ou des) mesure(s) du (ou des) paramètre(s) biométrique(s).

Par exemple, lorsque les moyens de génération d'ultrasons de la sonde comprennent au moins un transducteur agencé de sorte à générer des ultrasons focalisés de haute intensité, alors le modèle optimal de sonde est celui pour lequel la distance entre la zone à traiter et la zone de focalisation du transducteur est minimale lorsque ledit modèle optimal de sonde est mis en place sur l'oeil à traiter.

Des aspects préférés mais non limitatifs du dispositif selon l'invention sont les suivants :
- l'étape de réception comprend la réception d'une mesure de la largeur horizontale de l'iris passant par le centre de la pupille dite distance blanc à blanc de l'oeil ;
   l'utilisation d'une mesure de la largeur horizontale de l'iris permet de déterminer la sonde optimale à utiliser pour le traitement de l'oeil à partir d'un examen oculaire simple ne nécessitant l'utilisation que d'instruments rudimentaires tels qu'une règle,
- l'étape de détermination du modèle optimal de sonde comprend les sous-étapes suivantes :
   ∘ Si la distance blanc à blanc est inférieure à un premier seuil, par exemple inférieure à 11 millimètres, alors on choisit un premier modèle de sonde,
   ∘ Si la distance blanc à blanc est comprise entre le premier seuil et un deuxième seuil, par exemple comprise entre 11 et 12 millimètres, on choisit un deuxième modèle de sonde,
   ∘ Si la distance blanc à blanc est supérieure au deuxième seuil, par exemple supérieure à 12 millimètres, on choisit un troisième modèle de sonde,
   les premier, deuxième et troisième modèles de sondes comprenant des anneaux de dimensions différentes ;
- l'étape de réception comprend la réception d'une mesure de la longueur axiale de l'oeil;
   l'utilisation d'une mesure de la longueur axiale de l'oeil permet de déterminer la sonde optimale à utiliser pour le traitement de l'oeil à partir d'un examen oculaire simple nécessitant un instruments oculaires courant à disposition de la majorité des praticiens,
- dans lequel le paramètre biométrique de l'oeil est choisi parmi :
   ∘ la largeur horizontale de l'iris passant par le centre de la pupille, et/ou
   ∘ la longueur axiale de l'oeil
   le modèle optimal de sonde étant déterminé en fonction de la largeur horizontale de l'iris passant par le centre de la pupille, et/ou de la longueur axiale de l'oeil.
   l'utilisation combinée d'une mesure de la longueur axiale de l'oeil et d'une mesure de la largeur horizontale de l'iris permet d'affiner la précision dans la détermination du modèle optimal de sonde.
- l'étape de détermination du modèle optimal de sonde comprend les sous-étapes suivantes :
   ∘ Si la longueur axiale de l'oeil est inférieure à une première valeur, par exemple inférieure à 23 millimètres, alors on choisit un premier modèle de sonde,
   ∘ Si la longueur axiale est comprise entre la première valeur et une deuxième valeur, par exemple comprise entre 23 et 25 millimètres, alors on choisit un deuxième modèle de sonde,
   ∘ Si la longueur axiale est supérieure à la deuxième valeur, par exemple supérieure à 25 millimètres, on choisit un troisième modèle de sonde,
   les premier, deuxième et troisième modèles de sondes comprenant des anneaux de dimensions différentes ;
- l'étape de réception comprend la réception d'une mesure de la distance blanc à blanc de l'oeil et la réception d'une mesure de la longueur axiale de l'oeil, l'étape de détermination du modèle optimal de sonde comprenant les sous-étapes suivantes :
   ∘ Si la distance longueur axiale de l'oeil est inférieure à une quatrième valeur, par exemple inférieure à 22 millimètre, alors on choisit un premier modèle de sonde,
   ∘ Si la longueur axiale de l'oeil est comprise entre la première et la quatrième valeur, par exemple comprise entre 22 millimètres et 23 millimètres, et si la distance blanc à blanc est inférieure au premier seuil, par exemple supérieure à 11 millimètres, alors on choisit un premier modèle de sonde,
   ∘ Si la longueur axiale de l'oeil est comprise entre la première et la quatrième valeur, par exemple comprise entre 22 millimètres et 23 millimètres et si la distance blanc à blanc est supérieure au premier seuil, par exemple supérieure à 11 millimètres, alors on choisit un deuxième modèle de sonde,
   ∘ Si la longueur axiale est comprise entre la première valeur et une cinquième valeur, par exemple comprise entre 23 et 24.5 millimètres, alors on choisit un deuxième modèle de sonde,
   ∘ Si la longueur axiale de l'oeil est comprise entre la cinquième valeur et une sixième valeur, par exemple comprise entre 24.5 millimètres et 25.5 millimètres et si la distance blanc à blanc est inférieure au deuxième seuil, par exemple inférieure à 12 millimètres, alors on choisit un deuxième modèle de sonde,
   ∘ Si la longueur axiale de l'oeil est comprise entre la cinquième valeur et une sixième valeur, par exemple comprise entre 24.5 millimètres et 25.5 millimètres et si la distance blanc à blanc est supérieure au deuxième seuil, par exemple supérieure à 12 millimètres, alors on choisit un troisième modèle de sonde,
   ∘ Si la longueur axiale est supérieure à la sixième valeur, par exemple supérieure à 25.5 millimètres, on choisit un troisième modèle de sonde,
   les premier, deuxième et troisième modèles de sondes comprenant des anneaux de dimensions différentes.
- le procédé comprend en outre les étapes suivantes :
   ∘ réception d'une image représentative du segment antérieur de l'oeil telle qu'une image par biomicroscopie ultrasonore,
   ∘ détermination de la région contenant les corps ciliaires dans l'image représentative du segment antérieur de l'oeil,
   ∘ superposition de l'image représentative du segment antérieur de l'oeil à une pluralité d'images gabarit, éventuellement après une étape de mise à l'échelle des images, de sorte à obtenir une pluralité d'images superposées, chaque image gabarit représentant au moins la zone de focalisation des ultrasons pour un modèle de sonde respectif,
   ∘ estimation de la distance entre la zone de focalisation et la région contenant les corps ciliaires,
   ∘ choix du modèle de sonde pour lequel la distance entre la zone de focalisation et la région contenant les corps ciliaires est minimale.
   Le fait d'utiliser une image par biométrie ultrasonore en plus du (ou des) paramètre(s) biométrique(s) permet d'améliorer la précision dans le choix du modèle optimal de sonde.

L'homme du métier appréciera que les dimensions indiquées ci-dessus en référence aux différents paramètres biométriques (11, 12, 22, 23, 24, 24.5, 25.5 etc.) sont à prendre en compte avec une marge de l'ordre de plus ou moins 0.2 (voir 0.4) et qui correspond à l'incertitude de mesure.

L'invention concerne également un produit programme d'ordinateur comprenant des instructions de code programme enregistré sur un support utilisable dans un ordinateur, **caractérisé en ce qu'il** comprend des instructions pour la mise en oeuvre du procédé décrit ci-dessus.

L'invention concerne également un système pour la détermination d'un modèle de sonde de traitement d'une pathologie oculaire parmi une pluralité de modèles de sondes différents, chaque sonde comportant un anneau incluant une extrémité proximale destinée à être en contact avec un oeil d'un patient et une extrémité distale destinée à recevoir des moyens de génération d'ultrasons, le système comprenant un calculateur programmé pour :
- recevoir au moins une mesure d'un paramètre biométrique de l'oeil,
- déterminer un modèle optimal de sonde en fonction de chaque paramètre biométrique.

### BREVE DESCRIPTION DES DESSINS

D'autres avantages et caractéristiques ressortiront mieux de la description qui va suivre de plusieurs variantes d'exécution, données à titre d'exemples non limitatifs, à partir des dessins annexés sur lesquels :
- les figures 1 et 2 illustrent un mode de réalisation du dispositif décrit dans WO 2009/103721 ;
- les figures 3 à 7 illustrent différentes variantes du procédé selon l'invention,
- la figure 8 et 9 illustrent des images gabarit et finale.

### DESCRIPTION DETAILLEE DE L'INVENTION

En référence aux figures 1 et 2, on a illustré un mode de réalisation du dispositif de traitement d'une pathologie oculaire décrit dans WO 2009/103721.

Le dispositif comprend une sonde composée :
- d'un anneau 1,
- de moyens de génération d'ultrasons 2 pour générer des ultrasons focalisés.

Ce dispositif permet le traitement du glaucome en un seul geste.

### Anneau

L'anneau 1 consiste en un tronc de cône ouvert aux deux extrémités. La petite base 11 du tronc de cône constitue l'extrémité proximale de l'anneau 1, et la grande base 12 constitue l'extrémité distale de l'anneau 1.

L'anneau 1 permet un positionnement adéquat et constant des moyens de génération d'ultrasons 2, aussi bien pour le centrage que pour la distance par rapport à la sclère desdits moyens de génération d'ultrasons 2.

L'extrémité proximale 11 est destinée à venir en contact avec un oeil 4 d'un patient. L'extrémité distale 12 de l'anneau 1 est destinée à recevoir les moyens de génération d'ultrasons 2.

En référence à la figure 1, l'extrémité proximale 11 du tronc de cône comprend une bride annulaire externe 13 apte à être appliquée sur la surface externe de l'oeil 4, à environ 2 mm du limbe, le limbe étant la jonction entre la cornée et le sclérotique.

Le bord proximal 11 du tronc de cône comporte également une gorge annulaire 14 reliée à un dispositif d'aspiration 5 par au moins une ouverture traversant le tronc de cône 1 et débouchant dans la gorge annulaire 14. Avantageusement, le dispositif d'aspiration 5 peut être contrôlé par une unité de commande 6.

Il est évident que le dispositif d'aspiration 5 peut être indépendant sans sortir du cadre de l'invention.

Le principe de fonctionnement du dispositif d'aspiration 5 est le suivant. Le tronc de cône 1 est appliqué sur l'oeil 4 du patient et le dispositif d'aspiration 5 est activé. Ceci induit la production d'une dépression dans la gorge annulaire 14 qui entraine une déformation de la conjonctive de l'oeil 4, cette déformation formant un joint torique dans la gorge annulaire 14.

Le tronc de cône 1 est alors étroitement lié à l'oeil 4 de sorte que le tronc de cône 1 suivra les micromouvements de l'oeil 4 au cours de la durée du traitement. Ceci permet d'assurer un maintien de la position centrée de l'appareil sur l'axe visuel.

### Moyens de générations d'ultrasons

Les moyens de génération d'ultrasons 2 permettent de générer une énergie ultrasonore. Les moyens de génération d'ultrasons comportent une couronne cylindrique 21 sur laquelle sont agencés un (ou plusieurs) transducteur(s) 22 adaptés pour générer des ultrasons.

Dans certains modes de réalisation, le profil du (ou des) transducteur(s) est adapté pour permettre l'orientation et la focalisation des ultrasons en un point donné. Dans d'autres modes de réalisation, le (ou les) transducteur(s) est (sont) associé(s) à un (ou plusieurs) réflecteur(s) permettant de réfléchir, orienter et focaliser les ultrasons générés en un point donné.

Les moyens de génération d'ultrasons 2 sont reliés à l'unité de commande 6. Celle-ci comporte un générateur de salve et des moyens pour spécifier les paramètres de la salve comme la fréquence, la puissance et la durée de chaque rafale, etc.

Le générateur de salve comprend au moins un générateur de signal sinusoïdal à une fréquence déterminée comprise entre 5 et 25 MHz, et de préférence entre 19 et 22 MHz, un amplificateur et un compteur électrique.

### Généralités relatives à l'utilisation du dispositif illustré aux figures 1 et 2

L'objectif du dispositif décrit dans WO 2009/103721 est de détruire de manière sélective une partie des corps ciliaires et d'épargner les structures adjacentes. Pour ce faire, le dispositif décrit dans WO 2009/103721 est basé sur la génération d'ultrasons de haute intensité focalisés sur les corps ciliaires. Cette focalisation des ultrasons en un point donné est obtenue en adaptant les dimensions et l'orientation de l'anneau et des moyens de génération d'ultrasons.

Comme les dimensions de l'oeil humain peuvent varier d'un individu à l'autre, les inventeurs du dispositif décrit dans WO 2009/103721 proposent différentes dimensions de sondes (anneau + moyens de générations d'ultrasons) pour permettre un positionnement optimal du point de focalisation des ultrasons au niveau des corps ciliaires de l'oeil à traiter.

Ainsi, les inventeurs du dispositif WO 2009/103721 proposent quatre modèles différents de sondes composées chacune d'un anneau et d'une couronne afin de répondre à la variabilité anatomique interindividuelle :
- un premier modèle dans lequel l'anneau a un diamètre de 10 millimètres,
- un deuxième modèle dans lequel l'anneau a un diamètre de 11 millimètres,
- un troisième modèle dans lequel l'anneau a un diamètre de 12 millimètres,
- un quatrième modèle dans lequel l'anneau a un diamètre de 13 millimètres.

Pour ces quatre modèles de sondes, les dimensions et l'orientation des moyens de génération d'ultrasons sont adaptées pour permettre un positionnement optimal de la zone de focalisation au niveau du corps ciliaire à traiter.

Un but de l'invention est de permettre de déterminer le modèle optimal de sonde à utiliser pour traiter un patient en fonction des dimensions de son oeil.

Dans les modes de réalisation de l'invention présentés dans la suite, on supposera que la détermination du modèle de sonde optimal doit se faire parmi les trois modèles de sondes décrits précédemment. Toutefois, il est bien évident que l'invention peut être mise en oeuvre sur un plus petit ou un plus grand nombre de modèles de sondes différents.

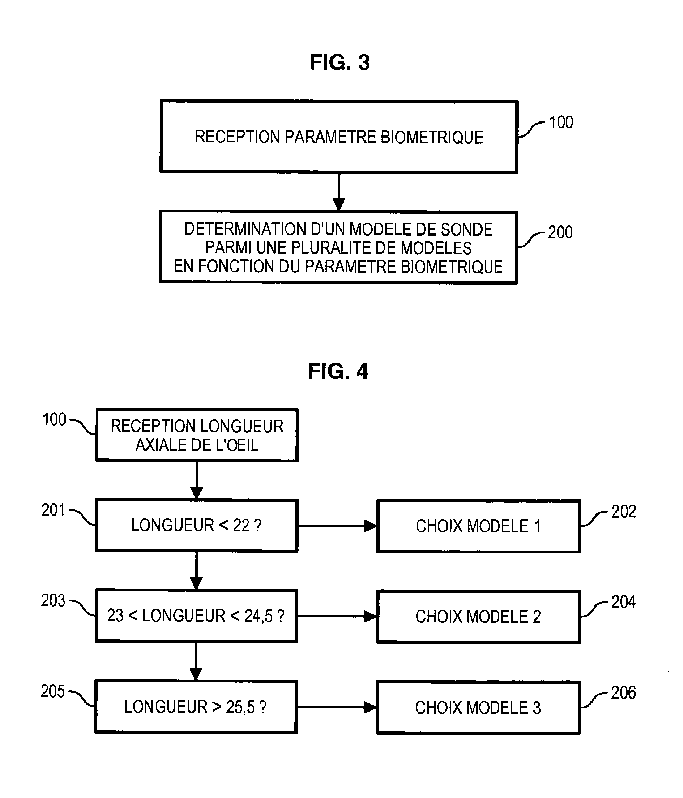

En référence à la figure 3, on a illustré un mode de réalisation du procédé selon l'invention.

Ce procédé comprend une première étape 100 de réception d'une (ou de plusieurs) mesure(s) d'un (ou de plusieurs) paramètre(s) biométrique(s).

Une deuxième étape 200 du procédé consiste à choisir parmi une pluralité de modèles de sondes différentes, la sonde dont les dimensions sont les mieux adaptées au patient. Ce choix est déterminé en fonction du (ou des) paramètre(s) biométrique(s) reçu(s) à l'étape précédente.

Ainsi, le procédé illustré à la figure 3 propose de déterminer le modèle optimal de sonde en fonction d'un (ou plusieurs) paramètre(s) biométrique(s) de l'oeil évalué(s) lors d'un examen clinique de routine (diamètre blanc à blanc, etc.), ou avec un examen para-clinique simple (longueur axiale, réfraction objective, etc.).

Ceci permet de réduire les coûts liés à la détermination du modèle optimal de sonde en s'affranchissant de l'utilisation d'un dispositif d'imagerie par biomicroscopie ultrasonore, appareil peu répandu du fait du coût élevé de celui-ci.

En référence à la figure 4, on a illustré un exemple d'étape de détermination d'une sonde optimale. Dans cet exemple, le paramètre biométrique pris en compte pour la détermination du modèle de sonde optimal parmi la pluralité de modèles de sondes est la longueur axiale de l'oeil.

Plus précisément :
- Si la longueur axiale de l'oeil est inférieure à 22 millimètres (étape 201), alors on choisit un premier modèle de sonde (étape 202),
- Si la longueur axiale est comprise entre 23 et 24.5 millimètres (étape 203), alors on choisit un deuxième modèle de sonde (étape 204),
- Si la longueur axiale est supérieure à 25.5 millimètres (étape 205), on choisit un troisième modèle de sonde (étape 206),

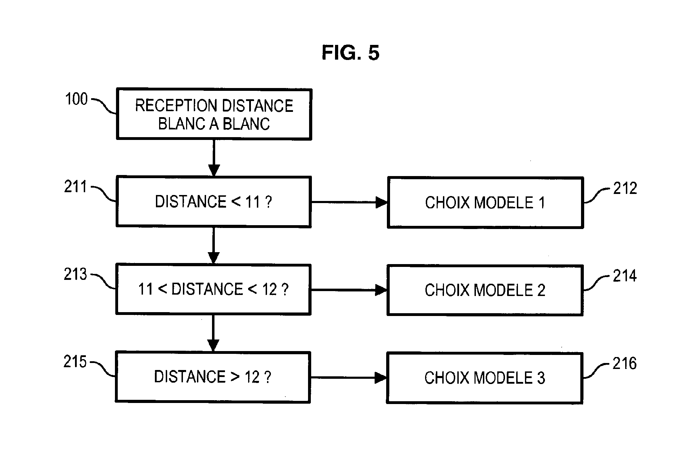

En référence à la figure 5, on a illustré un autre exemple d'étape de détermination d'une sonde optimale. Dans cet exemple, le paramètre biométrique pris en compte pour la détermination du modèle de sonde optimal parmi la pluralité de modèles de sonde est la largeur horizontale de l'iris dite distance blanc à blanc de l'oeil.

Plus précisément :
- Si la distance blanc à blanc est inférieure à 11 millimètres (étape 211), alors on choisit un premier modèle de sonde (étape 212),
- Si la distance blanc à blanc est comprise entre 11 et 12 millimètres (étape 213), alors on choisit un deuxième modèle de sonde (étape 214),
- Si la distance blanc à blanc est supérieure à 12 millimètres (étape 215), alors on choisit un troisième modèle de sonde (étape 216).

Ce procédé permet de déterminer un modèle de sonde optimal à partir d'une mesure facile à mettre en oeuvre, à savoir la distance blanc à blanc.

Pour améliorer le taux de validité du choix de sonde optimal parmi la pluralité de modèles de sonde, les deux paramètres biométriques précédemment cités - à savoir la longueur axiale de l'oeil et la distance blanc à blanc - peuvent être combinés.

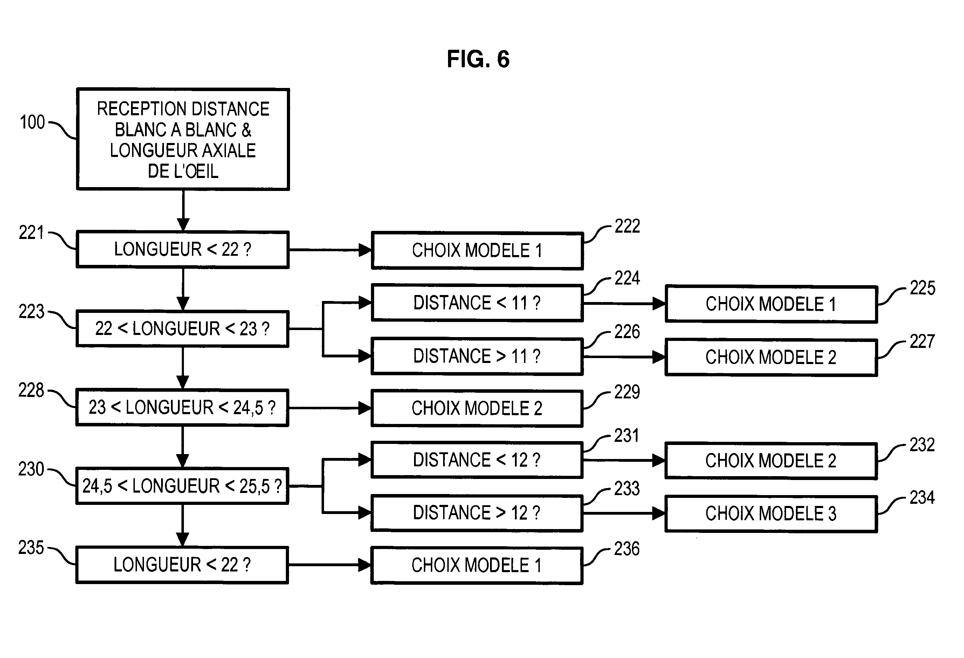

Plus précisément et en référence à la figure 6, l'étape de détermination d'une sonde optimale peut comprendre les sous-étapes suivantes :
- Si la distance longueur axiale de l'oeil est inférieure à 22 millimètres (étape 221), alors on choisit un premier modèle de sonde (étape 222),
- Si la longueur axiale de l'oeil est comprise entre 22 millimètres et 23 millimètres (étape 223) et si la distance blanc à blanc est inférieure à 11 millimètres (étape 224), alors on choisit un premier modèle de sonde (étape 225),
- Si la longueur axiale de l'oeil est comprise entre 22 millimètres et 23 millimètres (étape 223) et si la distance blanc à blanc est supérieure à 11 millimètres (étape 226), alors on choisit un deuxième modèle de sonde (étape 227),
- Si la longueur axiale est comprise entre 23 et 24.5 millimètres (étape 228), alors on choisit un deuxième modèle de sonde (étape 229),
- Si la longueur axiale de l'oeil est comprise entre 24.5 millimètres et 25.5 millimètres (étape 230) et si la distance blanc à blanc est inférieure à 12 millimètres (étape 231), alors on choisit un deuxième modèle de sonde (étape 232),
- Si la longueur axiale de l'oeil est comprise entre 24.5 millimètres et 25.5 millimètres (étape 230) et si la distance blanc à blanc est supérieure à 12 millimètres (étape 233), alors on choisit un troisième modèle de sonde (étape 234),
- Si la longueur axiale est supérieure à 25.5 millimètres (étape 235), on choisit un troisième modèle de sonde (étape 236).

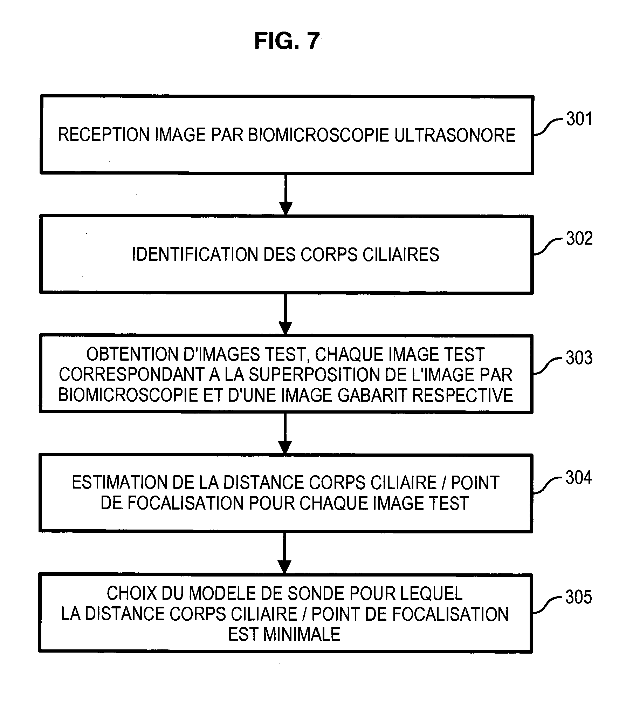

Dans le cas où l'on dispose d'une image par biomicroscopie ultrasonore de l'oeil du patient à traiter, il est possible de mettre en oeuvre le mode de réalisation du procédé illustré à la figure 7.

En référence à la figure 7, le procédé comprend les étapes suivantes :
- réception d'une image par biomicroscopie ultrasonore (étape 301),
- détermination de la région contenant les corps ciliaires dans l'image par biomicroscopie ultrasonore (étape 302),
- superposition de l'image dupliquée à une pluralité d'images gabarit afin d'obtenir une pluralité d'images finales, chaque image gabarit représentant au moins la zone de focalisation des ultrasons pour un modèle de sonde respectif (étape 303),
- estimation de la distance entre la zone de focalisation et la région contenant les corps ciliaires dans chaque image finale (étape 304),
- choix du modèle de sonde pour lequel la distance entre la zone de focalisation et la région contenant les corps ciliaires est minimale (étape 305).

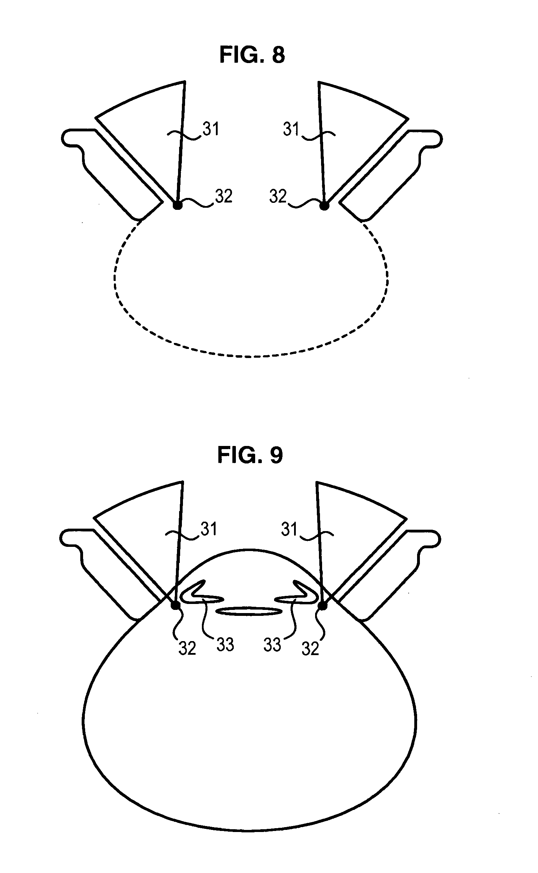

On a illustré un exemple d'image gabarit à la figure 9 et un exemple d'image finale à la figure 10. Chaque image gabarit peut comprendre une représentation partielle ou complète de la sonde - i.e. de l'anneau et de la couronne - ainsi qu'une représentation partielle ou complète du faisceau d'ondes ultrasonores 30 généré par les moyens de générations d'ultrasons 2. Ceci permet à l'utilisateur de vérifier visuellement pour chaque image finale correspondant à la superposition de l'image par biomicroscopie ultrasonore à une image gabarit respective si la position des corps ciliaire 33 de l'oeil à traiter coïncide avec le point de focalisation 32.

La détermination de la région contenant les corps ciliaires peut être mise en oeuvre par toute méthode de traitement connu de l'homme du métier telle qu'une méthode morpho mathématique à base de seuillage, etc.

Le procédé peut comprendre une étape de redimensionnement de l'image par biomicroscopie ultrasonore et de l'image gabarit afin que ces deux images soient à la même échelle. Cette étape peut être mise en oeuvre par toute technique connue de l'homme du métier.

De même, les étapes de superposition des images et d'estimation de la distance entre les corps ciliaires et le point de focalisation des ultrasons peuvent être mises en oeuvre par toute technique connue de l'homme du métier.

Le procédé décrit précédemment peut être mis en oeuvre dans un système de traitement comprenant un calculateur programmé pour exécuter les différentes étapes du procédé.

Le calculateur est par exemple un/des ordinateur(s), un/des processeur(s), un/des microcontrôleur(s), un/des micro-ordinateur(s), u n / d e s automate(s) programmable(s), un/des circuit(s) intégré(s) spécifique(s) d'application, d'autres circuits programmables, ou d'autres dispositifs qui incluent un ordinateur tel qu'une station de travail.

Le calculateur est couplée à une (ou plusieurs) mémoire(s) qui peut être intégrée au ou séparée du calculateur. La mémoire peut être une mémoire ROM/RAM du calculateur, un CD-ROM, une clé USB, une mémoire d'un serveur central. Cette mémoire peut permettre de stocker :
- les images par biomicroscopie ultrasonore, les images gabarit, et les images finales, ou encore
- le procédé de traitement mis en oeuvre par le calculateur.

L'homme du métier aura compris que de nombreuses modifications peuvent être apportées au dispositif décrit ci-dessus sans sortir matériellement des nouveaux enseignements présentés ici.
- Par exemple, dans la description qui précède, le dispositif et le procédé étaient présentées comme permettant de choisir un modèle optimal parmi trois modèles différents en fonction des dimensions de l'oeil à traiter. Il est bien évident que le système et le procédé peuvent permettre la détermination d'un modèle optimal parmi moins de trois modèles différents (par exemple deux), ou plus de trois modèles différents (quatre, cinq,... ou « n » modèle ayant des dimensions d'anneaux pouvant varier entre 1 et 100 millimètres pour le traitement d'enfant ou d'adultes). Par exemple, le procédé décrit précédemment peut être utilisé pour déterminer un modèle optimal parmi quatre modèles : un premier modèle dans lequel l'anneau a un diamètre de 10 millimètres,
- un deuxième modèle dans lequel l'anneau a un diamètre de 11 millimètres,
- un troisième modèle dans lequel l'anneau a un diamètre de 12 millimètres, ou
- un quatrième modèle dans lequel l'anneau a un diamètre de 13 millimètres.

Il est donc bien évident que les exemples qui viennent d'être donnés ne sont que des illustrations particulières en aucun cas limitatives.

Par exemple au lieu de recevoir une mesure d'un paramètre biométrique de l'oeil (longueur ou largeur de l'iris, etc.) pour déterminer un modèle optimal de sonde, le procédé peut comprendre la réception d'une valeur du paramètre biométrique correspondant à une moyenne calculée à partir d'une pluralité de mesures dudit paramètre biométrique.

En variante, le procédé peut comprendre :
- la réception d'une pluralité de mesures d'un paramètre biométrique,
- le calcul d'une valeur moyenne à partir de cette pluralité de mesures, et
- la détermination d'un modèle de sonde le plus adapté à l'oeil à traiter à partir de cette valeur moyenne calculée.

## Revendications

1. Procédé pour la détermination d'un modèle optimal de sonde de traitement d'une pathologie oculaire parmi une pluralité de modèles de sondes différents, chaque sonde comportant un anneau de forme conique incluant une extrémité proximale (11) destinée à être en contact avec un oeil (4) d'un patient et une extrémité distale (12) destinée à recevoir des moyens de génération d'ultrasons (2), **caractérisé en ce que** le procédé comprend :
- la réception d'au moins une mesure d'au moins un paramètre biométrique de l'oeil,
- la détermination du modèle optimal de sonde en fonction dudit et au moins un paramètre biométrique.

2. Procédé selon la revendication 1, dans lequel le paramètre biométrique de l'oeil est choisi parmi :
- la largeur horizontale de l'iris passant par le centre de la pupille, et/ou
- la longueur axiale de l'oeil
le modèle optimal de sonde étant déterminé en fonction de la largeur horizontale de l'iris passant par le centre de la pupille, et/ou de la longueur axiale de l'oeil.

3. Procédé selon la revendication 1, dans lequel l'étape de réception comprend la réception d'au moins une mesure de la largeur horizontale de l'iris passant par le centre de la pupille dite distance blanc à blanc de l'oeil.

4. Procédé selon la revendication 3, dans lequel l'étape de détermination du modèle optimal de sonde comprend les sous-étapes suivantes :
- Si la distance blanc à blanc est inférieure à 11 millimètres, alors on choisit un premier modèle de sonde,
- Si la distance blanc à blanc est comprise entre 11 et 12 millimètres, on choisit un deuxième modèle de sonde,
- Si la distance blanc à blanc est supérieure à 12 millimètres, on choisit un troisième modèle de sonde,
les premier, deuxième et troisième modèles de sondes comprenant des anneaux de dimensions différentes.

5. Procédé selon la revendication 1, dans lequel l'étape de réception comprend la réception d'au moins une mesure de la longueur axiale de l'oeil.

6. Procédé selon la revendication 5, dans lequel l'étape de détermination du modèle optimal de sonde comprend les sous-étapes suivantes :
- Si la longueur axiale de l'oeil est inférieure à 23 millimètres, alors on choisit un premier modèle de sonde,
- Si la longueur axiale est comprise entre 23 et 25 millimètres, alors on choisit un deuxième modèle de sonde,
- Si la longueur axiale est supérieure à 25 millimètres, on choisit un troisième modèle de sonde,
les premier, deuxième et troisième modèles de sondes comprenant des anneaux de dimensions différentes.

7. Procédé selon la revendication 1, dans lequel l'étape de réception comprend la réception d'au moins une mesure de la distance blanc à blanc de l'oeil et la réception d'au moins une mesure de la longueur axiale de l'oeil, l'étape de détermination du modèle optimal de sonde comprenant les sous-étapes suivantes :
- Si la distance longueur axiale de l'oeil est inférieure à 22 millimètre, alors on choisit un premier modèle de sonde,
- Si la longueur axiale de l'oeil est comprise entre 22 millimètres et 23 millimètres et si la distance blanc à blanc est inférieure à 11 millimètres, alors on choisit un premier modèle de sonde,
- Si la longueur axiale de l'oeil est comprise entre 22 millimètres et 23 millimètres et si la distance blanc à blanc est supérieure à 11 millimètres, alors on choisit un deuxième modèle de sonde,
- Si la longueur axiale est comprise entre 23 et 24.5 millimètres, alors on choisit un deuxième modèle de sonde,
- Si la longueur axiale de l'oeil est comprise entre 24.5 millimètres et 25.5 millimètres et si la distance blanc à blanc est inférieure à 12 millimètres, alors on choisit un deuxième modèle de sonde,
- Si la longueur axiale de l'oeil est comprise entre 24.5 millimètres et 25.5 millimètres et si la distance blanc à blanc est supérieure à 12 millimètres, alors on choisit un troisième modèle de sonde,
- Si la longueur axiale est supérieure à 25.5 millimètres, on choisit un troisième modèle de sonde,
les premier, deuxième et troisième modèles de sondes comprenant des anneaux de dimensions différentes.

8. Procédé selon l'une quelconque des revendications 1 à 7, lequel comprend en outre les étapes suivantes :
- réception (301) d'une image représentative du segment antérieur de l'oeil
- détermination (302) de la région contenant les corps ciliaires dans l'image représentative du segment antérieur de l'oeil,
- superposition (303) de l'image représentative du segment antérieur de l'oeil à une pluralité d'images gabarit de sorte à obtenir une pluralité d'images superposées, chaque image gabarit représentant au moins la zone de focalisation des ultrasons pour un modèle de sonde respectif,
- estimation (304) de la distance entre la zone de focalisation et la région contenant les corps ciliaires pour chaque image superposée,
- choix (305) du modèle de sonde pour lequel la distance entre la zone de focalisation et la région contenant les corps ciliaires est minimale.

9. Produit programme d'ordinateur comprenant des instructions de code programme enregistré sur un support utilisable dans un ordinateur, **caractérisé en ce qu'il** comprend des instructions pour la mise en oeuvre du procédé selon l'une des revendications 1 à 8.

10. Système pour la détermination d'un modèle de sonde de traitement d'une pathologie oculaire parmi une pluralité de modèles de sondes différents, chaque sonde comportant une anneau incluant une extrémité proximale (11) destinée à être en contact avec un oeil (4) d'un patient et une extrémité distale (12) destinée à recevoir des moyens de génération d'ultrasons (2),
**caractérisé en ce que** le système comprend un calculateur programmé pour :
- recevoir au moins une mesure d'au moins un paramètre biométrique de l'oeil,
- déterminer un modèle optimal de sonde en fonction dudit et au moins un paramètre biométrique.

## Patentansprüche

1. Verfahren zum Bestimmen eines optimalen Sondenmodells zur Behandlung einer Augenerkrankung aus einer Mehrzahl von unterschiedlichen Wobei jede Sonde einen Ring mit konischer Form aufweist, der ein proximales Ende (11) enthält, das dazu bestimmt ist, mit einem Auge (4) eines Patientin in Kontakt zu stehen, sowie ein distales Ende (12), das dazu bestimmt ist, Ultraschallerzeugungsmittel (2) aufzunehmen,
**dadurch gekennzeichnet, dass** das Verfahren umfasst:
- das Aufnehmen zumindest einer Messung zumindest eines biometrischen Parameters des Auges,
- das Bestimmen des optimalen Sondenmodells in Abhängigkeit von dem zumindest einen biometrischen Parameter.

2. Verfahren nach Anspruch 1, wobei der biometrische Parameter des Auges ausgewählt ist aus:
- der horizontalen Breite der Iris, die durch das Zentrum der Pupille verläuft, und/oder
- der axialen Länge des Auges,
wobei das optimale Sondenmodell in Abhängigkeit von der horizontalen Breite der Iris, die durch das Zentrum der Pupille verläuft, und/oder von der axialen Länge des Auges bestimmt wird.

3. Verfahren nach Anspruch 1, wobei der Schritt des Aufnehmens die Aufnahme zumindest einer Messung der horizontalen Breite der Iris umfasst, die durch das Zentrum der Pupille verläuft und Weiß-Weiß-Abstand des Auges genannt wird.

4. Verfahren nach Anspruch 3, wobei der Schritt des Bestimmens des optimalen Sondenmodells die nachfolgenden Unterschritte umfasst:
- bei einem Weiß-Weiß-Abstand von unter 11 mm Auswählen eines ersten Sondenmodells,
- bei einem Weiß-Weiß-Abstand zwischen 11 und 12 mm Auswählen eines zweiten Sondenmodells 15,
- bei einem Weiß-Weiß-Abstand von über 12 mm Auswählen eines dritten Sondenmodells,
wobei das erste, das zweite und das dritte Sondenmodell Ringe mit unterschiedlichen Abmessungen aufweisen.

5. Verfahren nach Anspruch 1, wobei der Schritt des Aufnehmens die Aufnahme zumindest einer Messung der axialen Länge des Auges umfasst.

6. Verfahren nach Anspruch 5, wobei der Schritt des Bestimmens des optimalen Sondenmodells die nachfolgenden Unterschritte umfasst:
- bei einer axialen Länge des Auges von unter 23 mm Auswählen eines ersten Sondenmodells,
- bei einer axialen Länge zwischen 23 und 25 mm Auswählen eines zweiten Sondenmodells,
- bei einer axialen Länge von über 25 mm Auswählen eines dritten Sondenmodells, wobei das erste, das zweite und das dritte Sondenmodell Ringe mit unterschiedlichen Abmessungen aufweisen.

7. Verfahren nach Anspruch 1, wobei der Schritt des Aufnehmens die Aufnahme zumindest einer Messung des Weiß-Weiß-Abstands des Auges und die Aufnahme zumindest einer Messung der axialen Länge des Auges umfasst, wobei der Schritt des Bestimmens des optimalen Sondenmodells die nachfolgenden Unterschritte umfasst:
- bei einer axialen Länge des Auges von unter 22 mm Auswählen eines ersten Sondenmodells,
- bei einer axialen Länge des Auges zwischen 22 mm und 23 mm und bei einem Weiß-Weiß-Abstand von unter 11 mm Auswählen eines ersten Sondenmodells,
- bei einer axialen Länge des Auges zwischen 22 mm und 23 mm und bei einem Weiß-Weiß-Abstand von über 11 mm Auswählen eines zweiten Sondenmodells,
- bei einer axialen Länge zwischen 23 und 24,5 mm Auswählen eines zweiten Sondenmodells,
- brei einer axialen Länge des Auges zwischen 24,5 mm und 25,5 mm und bei einem Weiß-Weiß-Abstand von unter 12 mm Auswählen eines zweiten Sondenmodells,
- bei einer axialen Länge des Auges zwischen 24,5 mm und 25,5 mm und bei einem Weiß-Weiß-Abstand von über 12 mm Auswählen eines dritten Sendenmodells,
- bei einer axialen Länge von über 25,5 mm Auswählen eines dritten Sondenmodells, wobei das erste, das zweite und das dritte Sondenmodell Ringe mit unterschiedlichen Abmessungen aufweisen.

8. Verfahren nach einem der Ansprüche 1 bis 7, wobei es ferner die nachfolgenden Schritte umfasst:
- Aufnehmen (301) eines Bildes, das für den vorderen Augenabschnitt repräsentativ ist,
- Ermitteln (302) des Bereichs, der die Ziliarkörper in dem für den vorderen Augenabschnitt repräsentativen Bild enthält,
- Überlagern (303) des für den vorderen Augenabschnitt repräsentativen Bildes auf eine Mehrzahl von Schablonenbildern, so dass eine Mehrzahl von überlagerten Bildern erhalten wird, wobei jedes Schablonenbild zumindest den Ultraschall-Fokussierungsbereich für ein jeweiliges Sondenmodell darstellt,
- Abschätzen (304) des Abstands zwischen dem Fokusssierungsbereich und dem Bereich mit den Ziliarkörpern für jedes überlagerte Bild,
- Auswählen (305) des Sondenmodells, bei dem der Abstand zwischen dem Fokussierungsbereich und dem Bereich mit den Ziliarkörpern minimal ist.

9. Computerprogrammprodukt mit Programmcode-Anweisungen, das auf einem in Computern verwendbaren Träger aufgezeichnet ist, **dadurch gekennzeichnet, dass** es Anweisungen zum Durchführen des Verfahrens nach einem der Ansprüche 1 bis 8 enthält.

10. System zum Bestimmen eines Sondenmodells zur Behandlung einer Augenerkrankung aus einer Mehrzahl von unterschiedlichen Sondenmodellen, wobei jede Sonde einen Ring aufweist, der ein proximales Ende (11) enthält, das dazu bestimmt ist, mit einem Auge (4) eines Patientin in Kontakt zu stehen, sowie ein distales Ende (12), das dazu bestimmt ist, Ultraschallerzeugungsmittel (2) aufzunehmen,
**dadurch gekennzeichnet, dass** das System einen programmierten Rechner aufweist zum:
- Aufnehmen zumindest einer Messung zumindest eines biometrischen Parameters des Auges,
- Bestimmen eines optimalen Sondenmodells in Abhängigkeit von dem zumindest einen biometrischen Parameter.

## Claims

1. Method for determining an optimum probe model for treating an eye pathology from among a plurality of different probe models, each probe including a cone shaped ring including a proximal end (11) intended to be in contact with one eye (4) of a patient and a distal end (12) intended to receive means for generating ultrasonic waves (2),
**characterised in that** the method comprises:
- receiving at least one measurement of at least one biometric parameter of the eye,
- determining the optimum probe model according to said and at least one biometric parameter.

2. Method according to claim 1, wherein the biometric parameter of the eye is selected from:
- the horizontal width of the iris passing through the centre of the pupil, and/or
- the axial length of the eye
the optimum probe model being determined according to the horizontal width of the iris passing through the centre of the pupil, and/or the axial length of the eye.

3. Method according to claim 1, wherein the receiving step comprises the reception of at least one measurement of the horizontal width of the iris passing through the centre of the pupil, a so-called white to white distance of the eye.

4. Method according to claim 3, wherein the step for determining the optimum probe model comprises the following substeps:
- If the white to white distance is less than 11 millimetres, then a first probe model is selected,
- If the white to white distance is comprised between 11 and 12 millimetres, then a second probe model is selected,
- If the white to white distance is greater than 12 millimetres, then a third probe model is selected,
the first, second and third probe models comprising rings with different dimensions.

5. Method according to claim 1, wherein the reception step comprise the reception of at least one measurement of the axial length of the eye.

6. Method according to claim 5, wherein the step for determining the optimum probe model comprises the following substeps:
- If the axial length of the eye is less than 23 millimetres, then a first probe model is selected,
- If the axial length is comprised between 23 and 25 millimetres, then a second probe model is selected,
- If the axial length is greater than 25 millimetres, then a third probe model is selected,
the first, second and third probe models comprising rings with different dimensions.

7. Method according to claim 1, wherein the reception step comprises the reception of at least one measurement of the white to white distance of the eye and the reception of at least one measurement of the axial length of the eye, the step for determining the optimum probe model comprising the
- If the axial length distance of the eye is less than 22 millimetres, then a first probe model is selected,
- If the axial length of the eye is comprised between 22 millimetres and 23 millimetres and the if the white to white distance is less than 11 millimetres, then a first probe model is selected,
- If the axial length of the eye is comprised between 22 millimetres and 23 millimetres and if the white to white distance is greater than 11 millimetres, then a second probe model is selected,
- If the axial length is comprised between 23 and 24.5 millimetres, then a second probe model is selected,
- If the axial length of the eye is comprised between 24.5 millimetres and 25.5 millimetres and if the white to white distance is less than 12 millimetres, then a second probe model is selected,
- If the axial length of the eye is comprised between 24.5 millimetres and 25.5 millimetres and if the white to white distance is greater than 12 millimetres, then a third probe model is selected,
- If the axial length is greater than 25.5 millimetres, then a third probe model is selected,
the first, second and third probe models comprising rings with different dimensions.

8. The method according to any of claims 1 to 7, which further comprises the following steps:
- receiving (301) an image representative of the anterior segment of the eye,
- determining (302) the region containing the ciliary bodies in the image representative of the anterior segment of the eye,
- superposing (303) the representative image of the anterior segment of the eye to a plurality of template images so as to obtain a plurality of superposed images, each template image illustrating at least the focusing area of the ultrasonic waves for a respective probe model,
- estimating (304) the distance between the focusing area and the region containing the ciliary bodies for each superposed image,
- selecting (305) the probe model for which the distance between the focusing area and the region containing the ciliary bodies is minimum.

9. Computer program product comprising program code instructions recorded on a medium which may be used in a computer, **characterised in that** it comprises instructions for applying the method according to one of claims 1 to 8,

10. System for determining a probe model for treating an eye pathology from among a plurality of different probe models, each probe including a ring including a proximal end (11) intended to be in contact with one eye (4) of a patient and a distal end (12) intended to receive means for generating ultrasonic waves (2),
**characterised in that** the system comprises a programmed computer for:
- receiving at least one measurement of at least one biometric parameter of the eye,
- determining an optimum probe model depending on said and on at least one biometric parameter.
